# EUROPEAN PATENT SPECIFICATION

(11) **EP 1 642 983 B1**
(45) Date of publication and mention of the grant of the patent: **11.04.2007**
(21) Application number: 05077139.3
(22) Date of filing: 22.01.1992
(51) Int. Cl.: C12P 7/64, A23D 9/00, A23C 11/04, A61K 31/20, A23L 1/30

(54) **Arachidonic acid and methods for the production and use thereof**
Arachidonsäure und Verfahren zu ihrer Herstellung sowie Verwendung derselben
Acide arachidonique, ses procédés de production et d'utilisation

(30) Priority: 24.01.1991 US 645454
(43) Date of publication of application: 05.04.2006
(62) Divisional of application: 99204324.0
(73) Proprietor: MARTEK CORPORATION, Columbia, MD 21045 (US)
(72) Inventor: Kyle, David J., Catonsville, MD 21228 (US)
(74) Representative: Mercer, Christopher Paul

(56) References cited:
- EP-A2- 0 140 805
- EP-A2- 0 148 303
- JP-A- 1 196 255
- H. YAMADA ET AL.: "Biotechnological processes for production of poly-unsaturated fatty acids" JOURNAL OF DISPERSION SCIENCE AND TECHNOLOGY, vol. 10, no. 4&5, 1989, pages 561-579, XP000884958
- DATABASE WPI Section Ch, Week 9004 Derwent Publications Ltd., London, GB; Class B05, AN 90-026632 XP002131309 "Prepn. of oil and fat contg. highly unsatd. fatty acid - comprises culturing specific microbial strain which can produce arachidonic acid with oil and fat as carbon source" & JP 01 304892 A (SUNTORY LTD) 8 December 1989 (1989-12-08)
- PATENT ABSTRACTS OF JAPAN vol. 13, no. 227 (C-600) [3575], 25 May 1989 (1989-05-25) & JP 01 038007 A (LION CORP), 8 February 1989 (1989-02-08)

## Description

This invention relates to a method of providing arachidonic acid to an infant formula, and to infant formula containing arachidonic acid containing fungal oil.

Arachidonic acid (ARA) is a long chain polyunsaturated fatty acid (PUFA) of the omega-6 class (5,8,11,14-elcosatetraenoic acid, i.e., 20:4). ARA is the most abundant C₂₀ PUPA in the human body. It is particularly prevalent in organ, muscle and blood tissues, serving a major role as a structural lipid associated predominantly with phospholipids in blood, liver, muscle and other major organ systems. In addition to its primary role as a structural lipid, ARA also ia the direct precursor for a number of circulating eicosenoids such as prostaglandin E₂ (PGE₂), prostacyclin I₂ (PGI₂), thromboxane A₂ (TₓA₂), and leukotrienes B₄, (LTB₄) and C₄ (LTC₄). These eicosenoids exhibit regulatory effects on lipoprotein metabolism, blood rheology, vascular tone, leucocyte function and platelet activation.

Despite its importance to human metabolism, ARA cannot be synthesized in humans de novo. ARA is synthesized by the elongation and desaturation of linoleic acid (LOA), an essential fatty acid. This process requires the presence of the enzyme Δ6-desaturase, an enzyme present in the human body in low levels, Burre et al., Lipids, 25:354-356 (1990). Accordingly, most ARA must be provided in the diet, and this is especially important during times of very rapid body growth, such as infancy.

During the first year of its life, an infant can double or triple its weight. Consequently, elevated levels of dietary ARA are required. To satisfy this increased demand, human breast milk contains high levels of ARA. Sanders et al., Am. J. Clin. Nutr., 31:805-813 (1978). ARA is the most prevalent C₂₀ PUFA in breast milk. Of those mothers, especially vegetarians, who do breast-feed their infants, many would benefit from additional dietary ARA. However, many mothers do not breast feed their infants, or do not breast feed for the entire period of rapid infant growth, choosing instead to utilize an infant formula.

No commercial infant formulas known to Applicant contain ARA. U.S. Patent No. 4,670,285 (Clandinin et al.), discloses the infant's requirement for fatty acids including ARA. To provide these fatty acids, Clandinin et al. suggest a blend of egg yolk, fish oil or red blood cell phospholipids and vegetable oils as the fat component of a proposed infant.formula. However, fish oil contains high quantities of eicosapentaneoic acid . (EPA). EPA is known to depress ARA synthesis in infants. Carlson, et al., INFORM, 1:306 (1990).' Thus, it would be desirable to be able to provide ARA without also providing additional EPA. Furthermore, egg yolks contain a relatively low concentration of ARA, such that Clandinin et al.'s mixture is not economically viable.

Because ARA is present in animal, but not vegetable, oils, its production in commercial quantities has remained a desirable, but elusive, goal. Shinmen, et al., Microbiol. Biotech. 31:11-16 (1989), have reported the production of ARA by an isolated fungus, *Mortierella alpina*, using conventional stirred tank fermentation. (See also Japanese Patent 1,215,245 to Shinmen et al.). After culturing, the organisms are harvested, dried and their lipids extracted from the fungal biomass with an organic solvent and the lipids chemically (covalently) modified. For example, the lipid mixture is hydrolyzed or converted to ethyl esters and then combined with cyclodextrin prior to use as a dietary supplement. Shinmen et al. do not disclose or suggest the administration of unmodified microbial oils.

*Porphyridium cruentum*, a red microalgae, can be grown in ponds in large quantities and has a lipid content which can contain up to 40% ARA. Ahern, et al. Biotech. Bioeng. 25:1057-1070 (1983). Unfortunately, The ARA is primarily associated with galactolipids, a complex polar lipid not present in breast milk. Thus, not only is the total usable ARA produced a fraction of one percent of the biomass, but the form of the ARA is not suitable for use as an additive to infant formula without further modification.

U.S. Patent No. 4,870,011 (Suzuki et al.) discloses a method for obtaining lipids such as γ-linolenic acid from fungi of the genus Mortierella. The γ-linolenic acid is purified from the mixture of lipids contained in the fungi.

DE 3603000A1 (Milupa) discloses a highly polyunsaturated acid fat mixture and its use as the fat component of an infant formula. The fat mixture has a high content of ARA and docosahexanoic (DHA) acids in a ratio of 2.5:1 respectively, as well as a high content of cholesterol. Sources of the fatty acids are listed as being certain types of macroalgae, fish oils, organ fats from beef and pork or highly refined egg yolk oil. A source of the DHA and ARA is said to be macroalgae of the phaecophyte and rhodophyte types. There is no suggestion to use any microbes as a source of oil. Algal and fish oils also typically include EPA which depresses ARA synthesis in vivo. Additionally, highly refined egg yolk oil is not an economical source of ARA. Moreover, there is no disclosure therein of an ARA-concentrated additive for supplementing pre-existing infant formula.

JP-A-01196 255 (Suntory) disclose an infant formula supplemented with ARA and EPA, which may be produced by Mortierella, to resemble human milk. However, the need of maintaining a low content of EPA, while providing a high amount of ARA is not identified.

Accordingly, there remains a need for an economical, commercially feasible method of producing ARA, preferably without concomitant production of EPA, for use in an infant formula such that the ARA levels in the formula approximate those levels in human breast milk. It is an object of the present invention to satisfy that need.

### Summary of the Invention

This invention relates to the production and use of arachidonic acid containing fungal oil (ARASCO) and to infant formula containing such oils. The oil can be referred to as a single cell oil. Fungi are cultivated under oil-producing conditions, harvested and the oil extracted and recovered. The oil, without further chemical modification, can be used directly to provide supplemental ARA to newborn infants. Advantages of the invention include its ease of production, and high purity, and lack of detectable amounts of EPA.

### Detailed Description of the Preferred Embodiment of the Invention

The present invention succeeds in providing an economical source of arachidonic acid (ARA). In one embodiment, this invention relates to a method of providing an arachidonic acid-containing fungal oil (ARASCO) which is substantially free of eicosapentaneoic acid (EPA) to an infant formula. As used herein, "substantially free" means that the EPA is present in less than about one fifth of the amount of ARA in the oil. This oil, a single cell.oil, can be administered directly, in an unmodified form. As used herein "unmodified" means that the chemical properties of the fatty acids, or the oils themselves, have not been covalently altered. Thus, for example, a temporary modification to the ARASCO or ARA which could be reversed following uptake of the oil would not be beyond the scope of this invention.

**Table 1. Fatty Acid Composition of Several Fungal Species.**

| | Fatty Acid | | | | | | | | total |
|---|---|---|---|---|---|---|---|---|---|
| species | 14:0 | 16:0 | 16.1 | 18:1 | 18:2 | 18:2 | 20:4 | 20:5 | fat |
| *Mortierella alpina* | -- | 8.2 | -- | 33.5 | 16.3 | 23.3 | 13.0 | -- | 3.0 |
| *Mortierella elongata* | 2.0 | 13.2 | -- | 26.6 | 11.9 | 13.2 | 13.8 | 2.4 | 4.0 |
| *Mortieralla isabellina* | 0.3 | 15.7 | 0.8 | 55.8 | 11.1 | 9.0 | -- | -- | 7.3 |
| *Saprolognia perasitica* | 7.4 | 19.1 | 1.9 | 6.3 | 24.5 | 12.5 | 10.5 | 10.5 | 9.3 |
| *Pythium catenulatum* | 6.5 | 9.9 | 10.3 | 21.2 | 18.5 | 3.5 | 13.4 | 10.9 | 5.0 |
| *Pythium coloratum* | 13.6 | 9.9 | -- | 14.7 | 10.9 | 2.5 | 24.3 | 21.7 | 2.2 |
| *Pythium gracile* | 14.7 | 9.1 | 2.2 | 14.8 | 12.6 | 3.6 | 22.1 | 5.7 | 4.5 |
| *Pythium irregulare* | 10.3 | 15.4 | 6.9 | 12.3 | 21.0 | 3.9 | 10.6 | 12.4 | 11.9 |
| *Pythium ultimum* | 9.5 | 16.7 | 10.5 | 17.1 | 20.7 | 1.3 | 9.0 | 6.9 | 13.3 |
| *Pythium insidiosum* | 9.5 | 11.4 | 12.1 | 1.0 | 8.3 | 9.3 | 31.9 | -- | 2.8 |

Of those fungal species which previously have had their fatty acids characterized, it has been found that most do not make ARA. Weete, J.D., Fungal Lipid Biochemistry, Plenum Press, N.Y. (1974). Of those species which do make ARA, many, including all previously characterized *Pythium* species, produce significant quantities of eicosapentaenoic acid (EPA) in addition.to ARA. Table 1 sets forth the fatty acid profile of species of fungi. As with fish oils, high EPA levels in dietary supplements result in a depression of the ability to form ARA from dietary linoleic acid (LOA). Accordingly, it is preferable to use a species which does not produce significant quantities of EPA, such as *Mortierella alpina*. This species is available commercially and is on deposit with the American Type Culture Collective in Rockville, Maryland, having accession number 42430.

One of the significant problems which the present invention overcomes, is the depression of ARA biosynthesis in infants caused by the presence of enhanced dietary levels of EPA. This problem can be corrected by providing ARA for use in infant formula at levels substantially similar to those found in human breast milk. Typically in human breast milk, the ratio of ARA:EPA is about 20:1 respectively. The present invention specifically contemplates a microbial oil extracted from Mortierella alpina wich provides a sufficient amount of ARA to overcome the negative effects of dietary EPA. Preferably, the use of the ARA-containing oil will result in an ARA : EPA ratio of at least about 5:1. More preferably, the ratio will be at least about 10:1 and, most preferably, it will be at least about 20:1. As can be seen, the higher the amount of ARA in the end product, with respect to the amount of EPA, the more desirable is the result.

In a process of the present invention, the Mortierella alpina fungi is cultivated under suitable ARA-containing oil producing cultivating conditions. In general, techniques of fungal cultivation are well known to those of skill in the art and those techniques can be applied to the present inventive process. For example, cultivation of an inoculating amount of fungus can occur in submerged culture in shake flasks. The flask is provided with a growth medium, seeded with fungal mycelium, and grown on a reciprocating shaker for about three to four days.

The composition of the growth medium can vary but always contains carbon and nitrogen sources. A preferred carbon source is glucose, amounts of which can range from about 10-100 grams glucose per liter of growth medium. Typically about 15 grams/liter are utilized for shaker flask culture. The amount can be varied depending upon the desired density of the final culture. Other carbon sources which can be used include molasses, high fructose corn syrup, hydrolyzed starch or any other low cost conventional carbon source used in fermentation processes . Suitable amounts of these carbon sources can readily be determined by those of skill in the art. Usually, additional carbon needs to be added during the course of the cultivation. This is because the organisms use so much carbon that adding it all in a batch mode could prove unwieldy.

Nitrogen typically is provided in the form of yeast extract at a concentration of from about 2 to about 15 grams extract per liter of growth medium. Preferably, about four grams per liter are provided. Other nitrogen sources can be used, including peptone, tryptone, cornsteep liquor, etc. The amount to be added of these sources can easily be determined by those of skill in the art. Nitrogen can be added in a batch mode, i.e. all at one time prior to cultivation.

After cultivation for 3-4 days at a suitable temperature, typically about 25-30°C, an amount of fungi has grown which is sufficient for use as an inoculum in a conventional stirred tank fermentor (STF). Such fermentors are known to those of skill in the art and are commercially available. Fermentation can be carried out in batch, fed-batch, or continuous fermentation modes. Preferably, the STF is equipped with a Rushton-type turbine impeller.

The fermentor is prepared by adding the desired carbon and nitrogen sources. For example, a 1.5 liter fermentor can be prepared by mixing about 50 grams of glucose and about 15 grams of yeast extract per liter of tap water. As previously discussed, other carbon or nitrogen sources or mixtures thereof can be used.

The reactor containing the nutrient solution should be sterilized by, for example, heating prior to inoculation. After cooling to about 30°C, the inoculum can be added, and cultivation initiated. Gas exchange is provided by air sparging. The air sparging rate can vary, but preferably is adjusted to from about 0.5 to about 4.0 VVM (volume of air per volume of fermentor per minute). Preferably the dissolved oxygen level is kept at from about 10% to about 50% of the air saturation value of the solution. Accordingly, adjustments in the sparge rate may be required during cultivation. Agitation is desirable. The agitation is provided by the impeller. Agitation tip speed preferably is set within the range of from about 50 cm/sec to about 500 cm/sec, preferably from about 100 to 200 cm/sec.

In general, the amount of inoculum can vary. Typically, from about 2% to about 10% by volume of inoculum can be used. Preferably, in a fermentor seed train about 5% by volume of inoculum can be used.

Nutrient levels should be monitored. When glucose levels drop below 5 g/l, additional glucose should be added. A typical cultivation cycle utilizes about 100 grams of glucose and about 15 grams of yeast extract per liter. It is desirable to deplete the nitrogen during the course of the cultivation as this enhances oil production by the M. alpina fungi.

Occasionally, the culture will produce an excessive quantity of foam. Optionally, an antifoaming agent, such as those known to those of skill in the art, e.g. Mazu 310^{®}, can be added to prevent foam.

The temperature of cultivation can vary. However, fungi which produce both ARA and EPA tend to produce less EPA and more ARA when cultivated at higher temperatures. Thus, when *Mortierella alpina* is cultivated at less than 18°C, it begins to produce EPA. Thus, it is preferable to maintain the temperature at a level which induces the preferential production of ARA. Suitable temperatures are typically from about 25°C to about 30°C.

Preferably, cultivation continues until a desired biomass density is achieved. A desirable biomass is about 25 g/l of the organism. Such a biomass typically is attained within 48-72 hours after inoculation. At this time, the organisms typically contain about 5-40% complex lipids, i.e. oil, of which about 10-40% is ARA, and can be harvested.

Harvesting can be done by any suitable method such as, for example, filtration, centrifugation, or spray drying. Because of lower cost, filtration may be preferred.

After harvesting, the mycelial cake can be extracted. The mycelial cake refers to the collection of biomass resulting after harvest. The cake can be loose or pressed, crumbled or uncrumbled. Optionally, the cake can have any residual water removed, as by vacuum drying or lyophilization, prior to extraction. If this option is selected, it is preferable to use nonpolar solvents to extract the ARA-containing oil. While any non-polar extract is suitable, hexane is preferred.

Alternatively, the wet cake (which typically contains about 30-50% solids) can be crumbled and extracted directly using polar solvents such as ethanol or isopropyl alcohol, or supercritical fluid extraction with solvents such as CO₂ or NO. Preferably, the cakes are crumbled prior to extraction. Advantageously, the present invention permits the economical use of supercritical fluid extraction techniques. McHugh, et al., Supercritical Fluid Extraction, Butterworth (1986). Such techniques are known to those of skill in the art and include those presently applied, for example, to decaffeinate coffee beans. While the yields from both wet and dry extractions are similar, wet extraction generally is a more economical process.

A preferable method of aqueous extraction involves mixing the mycelial biomass with the polar solvent isopropyl alcohol in a suitable reaction kettle. Such kettles are known. The use of three to six parts of solvent per part of biomass is desired. Most preferably, the mixing is done under nitrogen or in the presence of antioxidants to prevent the oxidation of the ARA in the lipid extract. As used herein "lipid extract", "oil", "lipid complex" and "fungal oil" are used interchangeably.

After extracting, the mixture can be filtered to remove the biomass from the solvent containing the lipid extract.

The solvent is separated from the lipid extract and also can be recovered for reuse, as by evaporation into a suitable collector, leaving what is referred to herein as the "crude oil." Use of isopropyl alcohol as the solvent desirably results in the removal of any residual water from the crude oil, as the evaporation removes the water/isopropyl alcohol azeotrope which has spontaneously formed.

While the crude oil can be used without further treatment, it also can be further purified. Processes such as those used in the preparation of lecithin from vegetable products, and known to those of skill in the art, can be used in this additional purification step. Such processes do not chemically or covalently modify the ARA-containing lipids or the ARA itself.

Yields vary, but typically 10-30 grams of triglyceride per 100 grams of dry mycelia can be obtained. Either the crude oil or the refined product can be used for administration to humans. Both shall be included within the definition of ARASCO as used herein.

An object of the invention is to provide human infant formulas, such that the concentration of ARA in such formula closely approximates the concentration of ARA in human breast milk. Table 2 compares the composition of the fatty acids in ARASCO with those in breast milk and infant formula lacking and containing ARASCO.

**Table 2. Fatty acid composition of fungal oil products and mother's milk**

| Patty Acid | ARASCO | Infant formula¹ | formula + oil | breast milk¹ |
|---|---|---|---|---|
| 8:0 | -- | 24.1 | 23.6 | 0.35 |
| 10:0 | -- | 17.7 | 17.3 | 1.39 |
| 12:0 | -- | 14.9 | 14.6 | 6.99 |
| 14:0 | 4.6 | 5.8 | 5.8 | 7.96 |
| 16:0 | 16.0 | 6.8 | 7.0 | 19.80 |
| 16:1 | 3.2 | 0.2 | 0.3 | 3.20 |
| 18:0 | -- | 2.3 | 2.3 | 5.91 |
| 18:1 | 26.4 | 10.0 | 10.3 | 34.82 |
| 18:2n6 | 9.9 | 17.4 | 17.3 | 16.00 |
| 18:3n3 | 4.1 | 0.9 | 1.0 | 0.62 |
| 20:1 | 2.2 | 0.1 | 0.14 | 1.10 |
| 20:2n6 | -- | -- | -- | 0.61 |
| 20:2n6 | 1.4 | -- | 0.03 | 0.42 |
| 20:4n6 | 32.0 | -- | 0.64 | 0.59 |
| 20:5n3 | -- | -- | -- | 0.03 |
| 22:1 | -- | -- | -- | 0.10 |
| 22:4n6 | -- | -- | -- | 0.21 |
| 22:5n6 | -- | -- | -- | 0.22 |
| 22:6n3 | -- | -- | -- | 0.19 |

| | | | | |
|---|---|---|---|---|
| ¹ Simopoulis, A., Omega-3 Fatty Acids in Health and Disease, pp.115-156 (1990) | | | | |

As can be seen, the amount of ARA present in the infant formula supplemented by ARASCO closely approximates the ARA levels in human breast milk. Additionally, the total fatty acid composition of the infant formula has not been significantly altered by the addition of the ARASCO. Between 50 to about 1000 mg of ARASCO per liter of infant formula can be used. The specific amount of ARASCO required depends upon the ARA content. This can vary from about 10 to about 50% of the fatty acids in the oil. However, typically the ARA content is about 30%. When the ARA content is about 30%, an especially preferred supplementation rate is about 600 to 700 mg of ARASCO per liter of infant formula. Such a rate dilutes the pre-existing fat cbmponents of an infant formula such as similar^{®} (Ross Laboratories, Columbus, Ohio) by only one part ARASCO to fifty parts formula oils. Preferably, the ARASCO is substantially free of EPA.

In the described process, the ARA-containing oil is predominantly triglyceride. This form of ARASCO is useful as an additive to infant formula. The oil from *M*. *alpina* is likely to be economical to produce.

The invention having been generally described, the following specific non-limiting example is set forth to further illustrate the invention.

### Example 1. Preparation of M. alpina lipid and addition to infant formula

*Mortierella alpina* (ATCC #42430) was grown in a 2 liter shake flask containing 1 liter of tap water and 20 grams of potato dextrose medium. The flask was under constant orbital agitation and was maintained at 25°C for seven days. After harvesting by centrifugation, the biomass was freeze dried yielding about 8 grams of lipid-rich mycelia. The mycelia was extracted using hexane with 1 liter of hexane per 200 grams of dry biomass at room temperature under continuous stirring for 2 hours and about 2.4g of crude oil resulted. This oil contains about 23% arachidonic acid and was added to the commercial formula Similac^{®} dropwise in concentrations of 1000 mg per liter.

## Claims

1. A method of providing arachidonic acid (ARA) to an infant formula wherein:
*Mortierella alpina* is cultivated under oil-producing conditions and harvested; the oil is extracted and recovered to produce an ARA-containing fungal oil (ARASCO) which is predominantly triglyceride and in which
(i) EPA is present in less than one fifth of the amount of ARA in the ARASCO and
(ii) the ARA content is from 10 to 50% of the fatty acids in the ARASCO; and
said infant formula is supplemented with between 50 to 1000 mg of unmodified ARASCO per liter of infant formula.

2. The method according to claim 1, wherein the ARA:EPA ratio in the infant formula supplemented with the ARASCO is at least 5:1.

3. The method according to claim 2, wherein the ARA:EPA ratio in the infant formula supplemented with the ARASCO is at least 10:1.

4. The method according to claim 3, wherein the ARA:EPA ratio in the infant formula supplemented with the ARASCO is at least 20:1.

5. The method according to any preceding claim, wherein the ARASCO is refined.

6. The method according to any one of claims 1 to 5, wherein the harvesting results in a mycelial cake, and wherein the cake has any residual water removed prior to extraction.

7. The method according to claim 6, wherein a nonpolar solvent is used to extract the ARASCO.

8. The method according to claim 7, wherein the nonpolar solvent is hexane.

9. The method according to any preceding claim, wherein the harvesting results in a mycelial cake, and wherein the cake is loose or pressed, crumbled or uncrumbled.

10. The method according to any preceding claim, wherein the *Mortierella alpina* is cultivated at from 25°C to 30°C.

11. The method according to any preceding claim, wherein the growth medium contains glucose, molasses, high fructose corn syrup, or hydrolyzed starch.

12. The method according to any preceding claim, wherein the growth medium contains 10-100 grams of glucose per liter of growth medium.

13. The method according to any preceding claim, wherein carbon is added during the course of the cultivation.

14. The method according to any preceding claim, wherein the growth medium contains peptone, tryptone, or cornsteep liquor.

15. The method according to any preceding claim, wherein the growth medium contains yeast extract at a concentration of from 2 to 15 grams per liter of growth medium.

16. The method according to any preceding claim, wherein nitrogen is depleted during the course of the cultivation.

17. The method according to any preceding claim, wherein agitation is provided by an impeller.

18. Infant formula comprising between 50 to 1000 mg per liter of infant formula of an arachidonic acid (ARA) containing fungal oil, produced by *Mortierella alpina,* in which oil (i) EPA is present in less than one fifth of the amount of ARA in the oil, (ii) the ARA content is from 10 to 50% of the fatty acids in the oil, and (iii) wherein the oil is predominantly triglyceride and unmodified.

19. Infant formula according to claim 18, wherein the ARA:EPA ratio in the formula is at least 5:1

20. Infant formula according to claim 19, in which the ARA:EPA ratio in the formula is at least 10:1.

21. Infant formula according to claim 20, in which the ARA:EPA ratio in the formula is at least 20:1.

22. Infant formula according to any one of claims 18-21, wherein the oil is refined.

## Patentansprüche

1. Ein Verfahren zur Bereitstellung von Arachidonsäure (ARA) für Säuglingsnahrung, wobei:
*Mortierella Alpina* unter ölerzeugenden Bedingungen kultiviert und geerntet wird; das Öl extrahiert und wieder zurückgewonnen wird, um ein ARA-haltiges Pilzöl (ARASCO) herzustellen, das überwiegend ein Triglycerid ist und in dem
(i) EPA in weniger als ein Fünftel der Menge von ARA im ARASCO vorhanden ist und
(ii) der ARA-Gehalt 10 bis 50% der Fettsäuren im ARASCO beträgt; und
die genannte Säuglingsnahrung mit 50 bis 1000 mg unmodifiziertem ARASCO pro Liter Säuglingsnahrung ergänzt ist.

2. Das Verfahren nach Anspruch 1, wobei das Verhältnis von ARA:EPA in der mit ARASCO ergänzten Säuglingsnahrung mindestens 5:1 beträgt.

3. Das Verfahren nach Anspruch 2, wobei das Verhältnis von ARA:EPA in der mit ARASCO ergänzten Säuglingsnahrung mindestens 10:1 beträgt.

4. Das Verfahren nach Anspruch 3, wobei das Verhältnis von ARA:EPA in der mit ARASCO ergänzten Säuglingsnahrung mindestens 20:1 beträgt.

5. Das Verfahren nach einem der vorhergehenden Ansprüche, wobei das ARASCO raffiniert ist.

6. Das Verfahren nach einem der vorhergehenden Ansprüche 1 bis 5, wobei das Geerntete einen Myzelkuchen ergibt, und wobei dem Kuchen vor der Extrahierung das restliche Wasser entfernt wurde.

7. Das Verfahren nach Anspruch 6, wobei zur Gewinnung des ARASCO ein unpolares Lösungsmittel verwendet wird.

8. Das Verfahren nach Anspruch 7, wobei das unpolare Lösungsmittel Hexan ist.

9. Das Verfahren nach einem der vorhergehenden Ansprüche, wobei das Geerntete einen Myzelkuchen ergibt, und wobei der Kuchen locker oder gepresst, zerbröckelt oder nicht zerbröckelt ist.

10. Das Verfahren nach einem der vorhergehenden Ansprüche, wobei der *Mortierella Alpina* bei 25°C bis 30°C kultiviert wird.

11. Das Verfahren nach einem der vorhergehenden Ansprüche, wobei das Kultivierungsmedium Glucose, Melasse, fructosereichen Maissirup oder hydrolysierte Stärke enthält.

12. Das Verfahren nach einem der vorhergehenden Ansprüche, wobei das Kultivierungsmedium 10-100 Gramm Glucose pro Liter Kultivierungsmedium enthält.

13. Das Verfahren nach einem der vorhergehenden Ansprüche, wobei während des Verlaufes der Kultivierung Kohlenstoff zugegeben wird.

14. Das Verfahren nach einem der vorhergehenden Ansprüche, wobei das Kultivierungsmedium Pepton, Trypton oder Maisquellwasser enthält.

15. Das Verfahren nach einem der vorhergehenden Ansprüche, wobei das Kultivierungsmedium Hefeextrakt in einer Konzentration von 2 bis 15 Gramm pro Liter Kultivierungsmedium enthält.

16. Das Verfahren nach einem der vorhergehenden Ansprüche, wobei während des Verlaufes der Kultivierung der Stickstoff vermindert wird.

17. Das Verfahren nach einem der vorhergehenden Ansprüche, wobei Rühren durch einen Kreiselrührer vorgesehen ist.

18. Säuglingsnahrung, die zwischen 50 bis 1000 mg pro Liter Säuglingsnahrung eines arachidonsäurehaltigen Pilzöles aufweist, welches durch *Mortierella Alpina* erzeugt wird, in dessen Öl (i) EPA in weniger als ein Fünftel der Menge von ARA im Öl vorhanden ist, (ii) der ARA-Gehalt 10 bis 50% der Fettsäuren im Öl beträgt und (iii) wobei das Öl überwiegend ein Triglycerid ist und nicht modifiziert ist.

19. Säuglingsnahrung nach Anspruch 18, wobei das Verhältnis von ARA:EPA in der Zubereitung mindestens 5:1 beträgt.

20. Säuglingsnahrung nach Anspruch 19, wobei das Verhältnis von ARA:EPA in der Zubereitung mindestens 10:1 beträgt.

21. Säuglingsnahrung nach Anspruch 20, wobei das Verhältnis von ARA:EPA in der Zubereitung mindestens 20:1 beträgt.

22. Säuglingsnahrung nach einem der Ansprüche 18-21, wobei das Öl raffiniert ist.

## Revendications

1. Procédé de fourniture d'acide arachidonique (ARA) à une préparation pour nourrissons dans lequel : *Mortierella alpina* est cultivé dans des conditions de production d'huile et récolté ; l'huile est extraite et récupérée pour produire une huile fongique contenant de l'ARA (ARASCO) qui est de manière prédominante un triglycéride et dans laquelle :
(i) de l'EPA est présent à moins d'un cinquième de la quantité d'ARA dans l'ARASCO et
(ii) la teneur en ARA est de 10 à 50 % des acides gras dans l'ARASCO ; et
ladite préparation pour nourrissons est complémentée avec entre 50 et 1000 mg d'ARASCO non modifié par litre de préparation pour nourrissons.

2. Procédé selon la revendication 1, dans lequel le rapport ARA : EPA dans la préparation pour nourrissons complémenté avec l'ARASCO est d'au moins 5 : 1.

3. Procédé selon la revendication 2, dans lequel le rapport ARA : EPA dans la préparation pour nourrissons complémenté avec l'ARASCO est d'au moins 10 : 1.

4. Procédé selon la revendication 3, dans lequel le rapport ARA : EPA dans la préparation pour nourrissons complémenté avec l'ARASCO est d'au moins 20 : 1.

5. Procédé selon l'une quelconque des revendications précédentes, dans lequel l'ARASCO est raffiné.

6. Procédé selon l'une quelconque des revendications 1 à 5, dans lequel la récolte résulte dans un pain mycélial, et dans lequel le pain a une quelconque eau résiduelle éliminée avant l'extraction.

7. Procédé selon la revendication 6, dans lequel un solvant non polaire est utilisé pour extraire l'ARASCO.

8. Procédé selon la revendication 7, dans lequel le solvant non polaire est l'hexane.

9. Procédé selon l'une quelconque des revendications précédentes, dans lequel la récolte résulte dans un pain mycélial, et dans lequel le pain est lâche ou pressé, émietté ou non émietté.

10. Procédé selon l'une quelconque des revendications précédentes, dans lequel *Mortierella alpina* est cultivé à 25 à 30°C.

11. Procédé selon l'une quelconque des revendications précédentes, dans lequel le milieu de croissance contient du glucose, de la mélasse, un sirop de maïs à teneur en fructose élevée, ou de l'amidon hydrolysé.

12. Procédé selon l'une quelconque des revendications précédentes, dans lequel le milieu de croissance contient de 10 à 100 grammes de glucose par litre de milieu de croissance.

13. Procédé selon l'une quelconque des revendications précédentes, dans lequel du carbone est ajouté au cours de la culture.

14. Procédé selon l'une quelconque des revendications précédentes, dans lequel le milieu de croissance contient du peptone, du tryptone ou de l'eau de macération de maïs.

15. Procédé selon l'une quelconque des revendications précédentes, dans lequel le milieu de croissance contient un extrait de levure à une concentration de 2 à 15 grammes par litre de milieu de croissance.

16. Procédé selon l'une quelconque des revendications précédentes, dans lequel l'azote est épuisé au cours de la culture.

17. Procédé selon l'une quelconque des revendications précédentes, dans lequel l'agitation est fournie par une roue à ailettes.

18. Préparation pour nourrissons comprenant entre 50 et 1000 mg par litre de préparation pour nourrissons d'une huile fongique contenant de l'acide arachidonique (ARA), produite par *Mortierella alpina,* dans laquelle (i) l'EPA est présent à moins de un cinquième de la quantité d'ARA dans l'huile, (ii) la teneur en ARA est de 10 à 50 % des acides gras dans l'huile, et (iii) dans laquelle l'huile est de manière prédominante un triglycéride et non modifiée.

19. Préparation pour nourrissons selon la revendication 18, dans laquelle le rapport ARA : EPA dans la formule est d'au moins 5 : 1.

20. Préparation pour nourrissons selon la revendication 19, dans laquelle le rapport ARA : EPA dans la formule est d'au moins 10 : 1.

21. Préparation pour nourrissons selon la revendication 20, dans laquelle le rapport ARA : EPA dans la formule est d'au moins 20 : 1.

22. Préparation pour nourrissons selon l'une quelconque des revendications 18 à 21, dans laquelle l'huile est raffinée.
